# EUROPEAN PATENT SPECIFICATION

(11) **EP 2 620 166 B1**
(45) Date of publication and mention of the grant of the patent: **29.03.2017**
(21) Application number: 11826812.7
(22) Date of filing: 20.09.2011
(51) Int. Cl.: A61L 31/04, A61B 17/072, A61L 31/06, A61L 31/14

(54) **SUTURE REINFORCING MATERIAL BEING FOR USE IN AUTOMATIC SUTURING DEVICES AND CONTAINING HYDROPHILIC POLYMER**
NAHTVERSTÄRKUNGSMATERIAL MIT HYDROPHILEN POLYMEREN ZUR VERWENDUNG IN AUTOMATISCHEN NÄHVORRICHTUNGEN
MATÉRIAU DE RENFORCEMENT DE SUTURES POUR UNE UTILISATION DANS DES DISPOSITIFS DE SUTURE AUTOMATIQUE ET CONTENANT UN POLYMÈRE HYDROPHILE

(30) Priority: 21.09.2010 JP 2010210570
(43) Date of publication of application: 31.07.2013
(73) Proprietor: The Doshisha, Kyoto-shi, Kyoto 602-8580 (JP); Gunze Limited, Ayabe-shi, Kyoto 623-8511 (JP)
(72) Inventor: HAGIWARA, Akeo, Kyotanabe-shi Kyoto 610-0394 (JP); TSUJIMOTO, Hiroyuki, Kyotanabe-shi Kyoto 610-0394 (JP); HASHIMOTO, Ayumi, Kyotanabe-shi Kyoto 610-0394 (JP); KUWABARA, Masayoshi, Kyotanabe-shi Kyoto 610-0394 (JP); NAKASE, Yuen, Kyoto-shi Kyoto 606-8174 (JP); MORITA, Shinichiro, Ayabe-shi Kyoto 623-8512 (JP)
(74) Representative: Vossius & Partner Patentanwälte Rechtsanwälte mbB
(86) International application number: PCT/JP2011/071300
(87) International publication number: WO 2012/039367

(56) References cited:
- EP-A1- 2 340 785
- EP-A2- 2 008 595
- JP-A- H0 847 526
- JP-A- H10 226 732
- JP-A- 2003 019 196
- JP-A- 2003 126 235
- JP-A- 2009 256 391
- JP-A- 2009 502 364
- US-A1- 2006 084 930
- KOBAYASHI H. ET AL.: 'In vivo evaluation of a new sealant material on a rat lung air leak model' J. BIOMED. MATER. RES. vol. 58, no. 6, 2001, pages 658 - 665, XP002688478
- OTANI Y. ET AL.: 'Sealing effect of rapidly curable gelatin-poly(L-glutamic acid) hydrogel glue on lung air leak' ANN. THORAC. SURG. vol. 67, no. 4, 1999, pages 922 - 926, XP027155487
- MASAHARU OGAKI ET AL.: 'Fukubu Geka ni Okeru Kyushusei Hogo Hokyozai (Neoberu) no Rinsho Oyo, Suieki-Ro, Lymph Eki-Ro, Tanju Roshutsu To no Boshi o Mokuteki to shite' THE 42ND ANNUAL CONGRESS OF JAPANESE SOCIETY FOR ABDOMINAL EMERGENCY MEDICINE PROGRAM SHOROKU 2006, page 288, VP-2, XP008169659

## Description

### Technical Field

The present invention relates to a suture reinforcement material suitable for use in an automatic suturing device used in surgical operations, etc.

### Background Art

Heretofore, a technique of simultaneously performing suturing and dissection of lungs, tubular organs, or the like, has widely been used in surgical operations. To perform this technique, a so-called automatic suturing device of a stapler-type containing a number of staples (sometimes referred to as an "automatic suturing and dissecting device") has been used (Non-Patent Document 1). Further, an automatic anastomosis device may sometimes be used to anastomose a tubular tissue such as transected intestine.

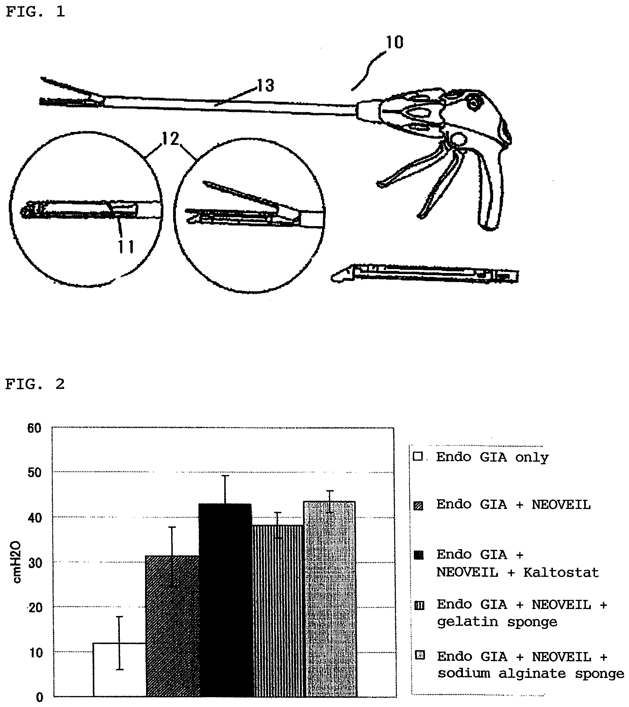

An automatic suturing device comprises (1) a dissecting member including a cutting knife for dissecting a biological tissue by sliding the cutting knife in the longitudinal direction on a center rail; and (2) a suturing member provided with staples for suturing a biological tissue. The staples are arranged generally in two or three lines at both ends of the center rail of the suturing member. In general, the suturing member further comprises a cartridge member containing staples and comprises a frame member having a staple-receiving groove. An automatic suturing device can perform suturing and dissection in one continuous operation by simultaneously or successively operating the suturing member and the dissecting member. Fig. 1 shows an example of an automatic suturing device. Fig. 1 shows a cutting knife 11, a cartridge member 12, and a frame member 13.

Staples are originally contained in the cartridge member, and are in a U shape inside the cartridge member. As the device is operated (fired), each staple is formed into a B shape to suture the edges of biological tissue. The staples are generally made of titanium, titanium alloy, or the like, which are less likely to cause rejection.

However, in the treatment using an automatic suturing device, tissue tearing would sometimes occur in the sutured region (suture site). Such a problem has been noticeable in the treatment of, in particular, vulnerable biological tissues, such as lung, bronchus, liver, and gastrointestinal tract.

In order to reinforce a suture site to prevent tissue tearing, a method has been employed comprising performing suturing with a tubular sheet fit onto the suturing member of an automatic suturing device, so that a biological tissue is sutured with the tubular sheet being placed between the staples and the biological tissue. Such a sheet is sometimes called a "suture reinforcement material for an automatic suturing device."

A suture reinforcement material for an automatic suturing device is attached to the suturing member by fitting it onto the cartridge member and frame member of the suturing member. Fig. 1 shows an example in which a suture reinforcement material for an automatic suturing device is attached to an automatic suturing device. In Fig. 1, the suture reinforcement materials for an automatic suturing device are attached to both the cartridge member 12 and the frame member 13.

When suturing is performed in a state where a suture reinforcement material for an automatic suturing device is fit onto the suturing member of an automatic suturing device, a biological tissue over which the suture reinforcement material for an automatic suturing device is placed can be sutured with staples. Staple suturing over the suture reinforcement material for an automatic suturing device reinforces the suture site. The suture reinforcement material for an automatic suturing device is sutured together with a biological tissue by means of staples, and at the same time, dissected together with the biological tissue by means of a cutting knife. As such, the biological tissue is ultimately dissected into two pieces with both of the suture sites being reinforced by means of the suture reinforcement material for an automatic suturing device.

A suture reinforcement material for an automatic suturing device is generally in a tubular shape, because it is attached by being fit onto the suturing member of an automatic suturing device. Heretofore, a material in which a stretch net and a sheet comprising a biodegradable and bioabsorbable material are joined together into a tubular shape has been used. When in use, the position of such a tubular-shaped suture reinforcement material for an automatic suturing device is adjusted so as to allow the biodegradable and bioabsorbable material sheet portion to be brought into contact with a biological tissue (Patent Documents 1 and 2). Also known is a suture reinforcement material comprising a fibrous biodegradable polyester layer and a porous hydrophilic polymer, i.e. collagen, that assists in stopping bleeding (Patent Document 3).

Although a known suture reinforcement material for an automatic suturing device has a certain effect (reinforcement effect) in terms of reinforcing a suture site to prevent tissue tearing from occurring in the suture site, further improvement could be made in terms of preventing air leakage or liquid leakage at the suture site.

### Citation List

### Patent Document

Patent Document 1: JP8-47526A
Patent Document 2: JP2001-70433A
Patent Document 3: EP 2008595 A2

### Non-Patent Document

Non-Patent Document 1: Sanfujinka naishikyoka shujutsu (Obstetrics and Gynecologic Endoscopic Surgery), January 5, 2001, Yoshiyuki Iwata, 71 pages

### Summary of Invention

### Technical Problem

An object of the present invention is to provide a suture reinforcement material for an automatic suturing device that is for use by an automatic suturing device and that is excellent in reinforcing effect.

### Solution to Problem

The inventors found that the risk of the occurrence of tissue tearing in a suture site can be greatly reduced when a suture reinforcement material for an automatic suturing device comprises a layer containing a hydrophilic polymer on a portion thereof, as defined in claim 1, to be brought into contact with a biological tissue. The inventors also found that surprisingly, application, in particular to a lung, can significantly reduce air leakage at the suture site. It was further found that such a suture reinforcement material for an automatic suturing device can also be used as a reinforcement material for an automatic anastomotic device.

Based on such findings, the inventors continued their research, and completed the present invention. The present invention is described below.

### Advantageous Effects of Invention

The suture reinforcement material for an automatic suturing device of the present invention can significantly reduce the risk of tissue tearing during a suturing operation that uses an automatic suturing device, compared to known suture reinforcement materials for an automatic suturing device.

Therefore, although application of an automatic suturing device to a lung, bronchus, liver, gastrointestinal tract, and other vulnerable biological tissue would be considered to be relatively difficult due to the risk of tissue tearing, techniques with low risk can be realized with respect to these biological tissues.

Fatal risk of air leakage in relation to, in particular, a lung can be remarkably reduced.

### Brief Description of Drawings

Fig. 1 shows an example of an automatic suturing device and an example where the suture reinforcement material for an automatic suturing device of the present invention is attached to an automatic suturing device.
Fig. 2 is a graph showing the results of measuring intrapulmonary pressure at which air leakage was observed when the suture reinforcement material for an automatic suturing device of the present invention ("Endo GIA + NEOVEIL + Kaltostat" (Example 1), "Endo GIA + NEOVEIL + sodium alginate sponge" (Example 2), or "Endo GIA + NEOVEIL + gelatin sponge" (Example 3)) was applied to a lung. The results of the Control Examples ("only Endo GIA" and "Endo GIA + NEOVEIL") are also shown therein.

### Explanation of reference numerals

- 10: Automatic Suturing Device
- 11: Cutting Knife
- 12: Cartridge Member
- 13: Frame Member

### Description of Embodiments

The suture reinforcement material for an automatic suturing device of the present invention comprises a sheet (A) containing at least one biodegradable and bioabsorbable aliphatic polyesters, polyester ethers, and copolymers thereof; and a layer (B) containing a hydrophilic polymer formed on at least one surface of the sheet (A). The hydrophilic polymer is alginic acid or alginate the suture reinforcement material for an automatic suturing device of the present invention further comprises a calcium ion source (C) , which is at least one calcium ion source, selected from the group consisting of polysaccharides and saccharic acids.

### 1. Sheet (A) containing a biodegradable and bioabsorbable material

Aliphatic polyesters, polyester ethers, and copolymers thereof may be used singly or in a combination of two or more.

Aliphatic polyesters are not particularly limited as long as they have biodegradable and bioabsorbable properties. Examples thereof include glycolic acid, lactic acid, ε-caprolactone, dioxanone, trimethylene carbonate, polyvalerolactone, and other polymers. Examples also include copolymers of at least two of these compounds.

Polyester ethers are not particularly limited as long as they have biodegradable and bioabsorbable properties. Examples thereof include poly-1,4-dioxanone-2-one, poly-1,5-dioxepane-2-one, and the like. Examples thereof also include copolymers of ethylene glycol and aliphatic polyester. Examples also include copolymers of at least two of these compounds. These may be used singly or in a combination of two or more.

Copolymers of aliphatic polyester and polyester ether are not limited as long as they have biodegradable and bioabsorbable properties. Examples thereof include a copolymer of at least one aliphatic polyester exemplified above and at least one polyester ether exemplified above.

As described above, when the suture reinforcement material for an automatic suturing device is used by attaching it to an automatic suturing device, the suture reinforcement material for an automatic suturing device is ultimately dissected by means of a cutting knife. This dissecting operation is performed by pressing the cutting knife of the dissecting member of the automatic suturing device onto the sheet-like suture reinforcement material for an automatic suturing device in the horizontal direction of the sheet. Therefore, if the suture reinforcement material for an automatic suturing device has insufficient strength, dissection cannot be satisfactorily performed, because the suture reinforcement material for an automatic suturing device warps when the cutting knife is pressed thereon. For this reason, the suture reinforcement material for an automatic suturing device is required to have strength to such an extent that warping does not occur. In general, a hydrophilic polymer-containing layer, which is another essential element of the suture reinforcement material for an automatic suturing device, often has insufficient strength. It is therefore preferable that the sheet containing a biodegradable and bioabsorbable material has strength to such an extent that such warping does not occur.

In relation to having strength to such an extent that warping does not occur, the sheet containing a biodegradable and bioabsorbable material preferably has a nonwoven fabric structure. Preferable examples of starting materials of the sheet containing a biodegradable and bioabsorbable material include, but are not particularly limited to, polyglycolic acid, polylactic acid, glycolic acid-ε-caprolactone copolymer, lactic acid-ε-caprolactone copolymer, and the like.

The sheet preferably has a thickness of, for example, 10 mm or less, and more preferably 0.1 to 3 mm. However, there is no limitation to the thickness.

### 2. Layer (B) containing a hydrophilic polymer

The sheet (A) has on at least a portion of at least one surface thereof a layer (B) containing a hydrophilic polymer.

The layer (B) containing a hydrophilic polymer may be formed on one surface or both surfaces of the sheet (A).

When the layer (B) is brought into contact with a biological tissue, water is added to the layer (B), allowing the hydrophilic polymer of the layer (B) to turn into a gel. This gel functions to fill the space generated between the staples and tissue in the suture site, preventing tissue tearing, which is prone to occur in a suture site. Therefore, in the sheet (A), at least the entire surface that is brought into contact with a biological tissue may have a layer containing a hydrophilic polymer. Alternatively, in the sheet (A), a surface that is brought into contact with a biological tissue may have, at least at a portion that is to be staple-sutured, a layer containing a hydrophilic polymer.

Alginates are more preferable compared to alginic acid. These may be used singly or in a combination of two or more as a hydrophilic polymer.

Examples of alginates include alkali metal salts and alkaline earth metal salts. Alkali metal salts are preferably used, and sodium salts are more preferably used.

The layer containing a hydrophilic polymer is preferably porous, as it turns into a gel in a more prompt manner. Such a porous body may be produced, for example, by freeze-drying a solution containing a hydrophilic polymer. A method for freeze-drying may comprise, for example, freezing the solution at -80°C, followed by drying with a vacuum freeze dryer.

A solution containing a hydrophilic polymer for use in the formation of a porous body by freeze-drying may be prepared by dissolving a hydrophilic polymer in a solvent; the dissolution concentration is not particularly limited.

The layer may have a thickness of, for example, 10 mm or less, but it is not limited to this thickness. The thickness is preferably 0.1 to 3 mm when operability is taken into consideration.

### 3. Calcium ion source (C)

The suture reinforcement material for an automatic suturing device of the present invention does not necessarily comprise a calcium ion source (C). For example, the calcium ion source (C) may be externally added to the layer (B). The mode thereof is not particularly limited. For example, a liquid containing calcium ions may be sprayed externally.

When the suture reinforcement material for an automatic suturing device comprises the calcium ion source (C), the gel strength of the layer (B) containing an alginic acid or a salt thereof improves, exerting a more excellent reinforcing effect in a suture site.

The calcium ion source (C) supplies calcium ions to the alginic acid or a salt thereof of the layer (B). When calcium ions are supplied to the alginic acid of the layer (B), the alginic acid in the layer (B) turns into a gel in a rapid manner.

The calcium ion source (C) may be arranged at any position on the suture reinforcement material for an automatic suturing device, as long as it can supply calcium ions to the alginic acid or a salt thereof of the layer (B). It is preferable that the calcium ion source (C) be in contact with the layer (B), contained in the layer (B), or mixed with the layer (B).

The calcium ion source is at least one type of calcium salt selected from the group consisting of polysaccharides and saccharic acids. Examples of polysaccharides include alginic acid, hyaluronic acid, pectin, and the like, with alginic acid being more preferable. Examples of saccharic acids include gluconic acid and the like, with gluconic acid being more preferable.

### 4. Suture reinforcement material for an automatic suturing device

Specific structures of the suture reinforcement material for an automatic suturing device of the present invention are described below. In any structure, the uppermost layer is used in such a manner that it faces the direction of a biological tissue.
(1) A structure in which the layer (B) is provided on the sheet (A);
(2) A structure in which the layer (B) is first provided on the sheet (A), and the calcium ion source (C) is further provided thereon;
(3) A structure in which the calcium ion source (C) is first provided on the sheet (A), and the layer (B) is further provided thereon; and
(4) A structure in which a mixture comprising the calcium ion source (C) and the layer (B) is provided on the sheet (A).

The suture reinforcement material for an automatic suturing device refers to a reinforcement material used for reinforcing a suture site to prevent the occurrence of tearing, etc., of the suture site, when an automatic suturing device (an automatic suturing and dissecting device) is used to suture a biological tissue.

The suture reinforcement material for an automatic suturing device of the present invention can be applied to any type of automatic suturing device. The automatic suturing device may be an automatic anastomotic device for thoracotomy, or an automatic suturing device for endoscopes. Examples of automatic suturing devices for thoracotomy include the Proximate (trademark), linear cutter, 55 mm, and Proximate (trademark), linear cutter, 75 mm (both produced by Ethicon Endo-Surgery, Inc.); the Multifire GIA (trademark), 60 cartridge, and Multifire GIA (trademark) 80 cartridge (both produced by United States Surgical Corporation); and the like.

Examples of automatic suturing devices for endoscopes include the ENDOPATH (trademark) Endocutter ETS45; ENDOPATH (trademark) Endocutter 60 ECHELON 60; and ENDOPATH (trademark) Endocutter EZ45 (all produced by Ethicon Endo-Surgery, Inc.); the ENDO GIA (trademark) II30 cartridge; ENDO GIA (trademark) II45 cartridge; and ENDO GIA (trademark) II60 cartridge (all produced by Covidien); and the like.

The suture reinforcement material for an automatic suturing device is used by previously fitting it onto the suturing member before a biological tissue is sutured and dissected by the automatic suturing device.

The suture reinforcement material for an automatic suturing device preferably has a tubular shape because it is attached to the suturing member by fitting it onto the suturing member. The tubular shape refers to a shape formed by combining both ends of a sheet-like material to join them with each other. The tubular shape may be cylindrical, prismatic, or planate.

In the entire tubular-shaped suture reinforcement material for an automatic suturing device, it is possible that only the surface to be in contact with biological tissue be formed from the suture reinforcement material for an automatic suturing device of the present invention, and other portions be formed from a well-known material.

When one sheet of a sheet-like material is used, the formation of the sheet-like material into a tubular shape can be done in such a manner, for example, that fabric having a size equal to the outer peripheral size of the suturing member is rolled up, followed by sewing, etc., the ends of the fabric. When two sheets of sheet-like materials are used, the formation of the sheet-like material into a tubular shape can be done in such a manner, for example, that the sheet-like materials are superimposed on each other, followed by sewing, etc., the ends thereof.

Examples of such sewing methods include a method of sewing the ends in parallel, a method of sewing in a manner such that the tip portion is tapered, a method of continuously sewing so as to form the tip portion into a bag, and other methods. The sewing method may be arbitrarily selected. The tip portion being tapered or being in a bag shape is advantageous for easy attachment to the suturing member.

As the thread used in the above-described sewing, sewing thread that is generally used for sewing a garment may be used as is. Considering the particularity of medical use, the sewing thread is preferably suture thread for surgery, such as a biodegradable and bioabsorbable suture thread comprising a polyglycolic acid, a copolymer of glycolic acid/lactic acid, a copolymer of lactic acid/caprolactone, or the like.

The size and shape of the suture reinforcement material for an automatic suturing device are suitably determined in detail according to the shape of the suturing member of the automatic suturing device to which the suture reinforcement material is to be applied.

### 5. Stretch knitted or woven fabric (D)

The suture reinforcement material for an automatic suturing device of the present invention does not necessarily comprise a stretch knitted or woven fabric (D). However, when the suture reinforcement material for an automatic suturing device is formed into a tubular shape, a part of the constituent material is preferably a stretch knitted or woven fabric (D) because this has the following advantages.

First, the stretchability allows easy attachment to the automatic suturing device. Second, after attachment thereof, the adhesion property increases and the occurrence of displacement is prevented. Third, the suture reinforcement material for an automatic suturing device can be put into a state of tension at the time of attachment, and dissection can thereby be easily and reliably performed.

In the stretch knitted or woven fabric (D), for example, rubber yarn, polyurethane-based elastic yarn, crimped yarn, textured yarn, or the like, is appropriately knitted or woven inside the structure. The fabric (D) has stretchability in vertical and horizontal directions. The structure thereof is not particularly limited. From the viewpoint of ease of cutting, ease of sewing at the time of the formation of a tubular shape, as well as the stability of the shape, particularly preferable examples include a powernet fabric in which a polyurethane yarn covered with a nylon yarn is warp knitted.

### 6. Method for producing a suture reinforcement material for an automatic suturing device

The suture reinforcement material for an automatic suturing device can be produced, for example, in the following manner. Each of the structures specifically described in "4. Suture reinforcement material for an automatic suturing device" is described below.

### (1) Structure in which the layer (B) is provided on the sheet (A)

The suture reinforcement material for an automatic suturing device can be produced by a method comprising step 1 of forming the layer (B) on at least one surface of the sheet (A).

The step of forming the layer (B) on the sheet (A) may comprise, for example, simply superimposing the layer (B) on the sheet (A). After the superimposition, sewing, adhesion, or the like, may be additionally carried out between (A) and (B), if necessary. It is also possible for a part of the layer (B) to be incorporated in the sheet (A).

As the thread used in the above-described sewing, a sewing thread that is generally used for sewing a garment may be used as is. Considering the particularity of medical use, the sewing thread is preferably suture thread for surgery, such as a biodegradable and bioabsorbable suture thread comprising a polyglycolic acid, a copolymer of glycolic acid/lactic acid, a copolymer of lactic acid/caprolactone, or the like.

When the layer (B) can be made into a liquid or semi-liquid state under certain conditions with the addition of an aqueous solution as necessary, the layer (B) can be formed on the sheet (A), for example, in the following manner. The sheet (A) is immersed in a liquid or semi-liquid containing the components of the layer (B). Alternatively, a liquid or semi-liquid containing the components of the layer (B) is applied to the sheet (A). Thereafter, the components of the layer (B) are converted into a solid state. Thereby, the layer (B) is formed on the sheet (A). For example, when sodium alginate is used as the layer (B), the addition of water turns sodium alginate into the state of an aqueous solution. This aqueous sodium alginate solution is applied to the sheet (A), and then freeze-dried to make the layer (B) porous on the sheet (A). In this manner, the layer (B) is eventually formed on the sheet (A). The sponge layer (B) may be formed only on one surface of the sheet (A), or the sponge layers (B) may be formed on both surfaces of the sheet (A) so that the sheet (A) is sandwiched between the sponge layers (B).

Examples of a freeze-drying method include a method comprising freezing at -80°C, followed by drying using a vacuum freeze dryer.

### (2) Structure in which the layer (B) is first provided on the sheet (A), and the calcium ion source (C) is further provided thereon

The suture reinforcement material for an automatic suturing device can be produced by a method comprising step 2-1 of forming the layer (B) on at least one surface of the sheet (A); and step 2-2 of further forming the calcium ion source (C) thereon.

The step of forming the calcium ion source (C) on the layer (B) may comprise, for example, simply superimposing the calcium ion source (C) on the layer (B). After the superimposition, sewing, adhesion, or the like, may be additionally carried out between (B) and (C), if necessary.

The sewing thread used in this sewing and the adhesive agent used in this adhesion may be those described above.

When the calcium ion source (C) can be made into a liquid or semi-liquid state under certain conditions with the addition of an aqueous solution as necessary, the calcium ion source (C) can be formed on the sheet (A), for example, in the following manner. The sheet (A) is immersed in a liquid or semi-liquid containing the components of the calcium ion source (C). Alternatively, a liquid or semi-liquid containing the components of the calcium ion source (C) is applied to the sheet (A). Subsequently, the components of the calcium ion source (C) are converted into a solid state. Thereby, the layer (B) can be formed on the sheet (A). Examples of a method for conversion into a solid include freeze-drying. Examples of a freeze-drying method include a method comprising freezing at a temperature of -80°C, followed by drying using a dryer.

### (3) Structure in which the calcium ion source (C) is first provided on the sheet (A), and the layer (B) is further provided thereon

The suture reinforcement material for an automatic suturing device can be produced by a method comprising step 3-1 of forming the calcium ion source (C) on at least one surface of the sheet (A); and step 3-2 of forming the layer (B) thereon.

The step of forming the calcium ion source (C) on the sheet (A) may comprise, for example, simply superimposing the calcium ion source (C) on the sheet (A). After the superimposition, sewing, adhesion, or the like, may be additionally carried out between (A) and (C), if necessary.

The sewing thread used in this sewing and the adhesive agent used in this adhesion may be those described above.

When either the calcium ion source (C) or the layer (B) can be made into a liquid or semi-liquid state under certain conditions with the addition of an aqueous solution as necessary, the formation of (C) or (B) can be made in the manner described above.

### (4) Structure in which a mixture comprising the layer (B) and the calcium ion source (C) is provided on the sheet (A)

The suture reinforcement material for an automatic suturing device can be produced by a method comprising step 4-1 of mixing the layer (B) with the calcium ion source (C); and step 4-2 of applying the resulting mixture to at least one surface of the sheet (A).

The step of mixing the layer (B) with the calcium ion source (C) may be performed in the following manner. When at least one of the layer (B) or the calcium ion source (C) can be formed into a liquid or semi-liquid state under a certain condition with the addition of an aqueous solution as necessary, the other one is immersed in this liquid or semi-liquid while the liquid or semi-liquid state is kept as is. Alternatively, this liquid or semi-liquid is added to the other to thereby mix them with each other. For example, when sodium alginate is used as the layer (B), the addition of water turns sodium alginate into the state of an aqueous solution. The calcium ion source (C) is immersed in this aqueous sodium alginate solution to be mixed therewith.

The above-described method may further include a step of forming the layer (B) into a porous body.

Examples of the step involving a porous body formation include the following. When the layer (B) can be formed into a liquid or semi-liquid state under certain conditions with the addition of an aqueous solution as necessary, the layer (B) is once formed into a liquid or semi-liquid state, and then freeze-dried to thereby be formed into a porous body. Examples of a freeze-drying method include a method comprising freezing at -80°C, followed by drying using a dryer.

### 7. Method of using suture reinforcement material for an automatic suturing device

The suture reinforcement material for an automatic suturing device is used by attaching it to an automatic suturing device to suture a biological tissue.

An automatic suturing device, the method for attaching the suture reinforcement material for an automatic suturing device to an automatic suturing device, suturing by means of an automatic suturing device, etc., are similar to those described in "4. Suture reinforcement material for an automatic suturing device." The descriptions therefor are thus omitted.

### Examples

The present invention is described below in more detail with reference to Examples. However, the scope of the present invention is not limited only to the following Examples.

### Example 1

### (1) Production of a suture reinforcement material for an automatic suturing device

The suture reinforcement material for an automatic suturing device of the present invention was produced in the following manner.

### • Preparation Method

(i) A sheet formed of a mixture of alginic acid calcium salt and alginic acid sodium salt ("Kaltostat" (trademark); Convatec Japan K.K.; hereinafter referred to as "alginic acid-Ca/Na salt sheet") was cut into a size of 3.5 X 7 cm.
(ii) A 2% sodium alginate solution was coated all around the cut alginic acid-Ca/Na salt sheet.
(iii) The coated alginic acid-Ca/Na salt sheet was subjected to freezing at -80°C and then drying using a freeze dryer to make it into a sponge (porosification).
(iv) A felt comprising polyglycolic acid ("NEOVEIL" (product name); GUNZE LIMITED) having almost the same size was superimposed on the alginic acid-Ca/Na salt sheet in a sponge form.

### (2) Suturing and dissection of a biological tissue using a suture reinforcement material for an automatic suturing device

The reinforcement material for an automatic suturing device of the present invention was used in the following manner to suture and dissect lung tissue of a dog.

### • Method of suturing and dissection

(i) The dog's chest was opened to expose the lung.
(ii) The lung was maintained at 20 cmH₂O for 2 minutes.
(iii) A felt comprising polyglycolic acid ("NEOVEIL" (product name); GUNZE LIMITED) was attached to an automatic suturing device for endoscopes ("Endo GIA" (trademark); Ethicon Endo-Surgery, Inc.) so as to cover the suturing member of the device.
(iv) The alginic acid-Ca/Na salt sheet in a sponge form produced in (1) above was attached to the suturing member of the automatic suturing device for endoscopes in such a manner that it was superimposed on the felt comprising polyglycolic acid.
(v) The right middle lobe was pinched with the automatic suturing device for endoscopes that was in the state of (iv), and maintained as is for 10 seconds.
(vi) The automatic suturing device for endoscopes was operated to perform suturing and dissection, and the lung was maintained as is for another 10 seconds.
(vii) The pressures 1, 2, and 3 minutes after the suturing and dissection were recorded.
(viii) After the completion of recording at each point, the lung was swollen, and the pressure at which air leakage started to occur was recorded in cmH₂O. Note that soapy water was poured over the lung, and the pressure at which foaming started to occur was measured in cmH₂O as the point at which air leakage started to occur.

### • Control Examples

The same procedures were performed, except that (iii) and (iv) were not conducted. This was regarded as Control Example A. Control Example A demonstrates a case where suturing and dissection were performed with nothing being attached to the suturing member of the automatic suturing device for endoscopes.

The same procedures were performed, except that (iv) was not conducted. This was regarded as Control Example B. Control Example B demonstrates a case where suturing and dissection were performed with only a felt comprising polyglycolic acid being attached to the suturing member of the automatic suturing device for endoscopes.

### • Results

Fig. 2 shows the results. In the Example where the alginic acid-Ca/Na salt sheet in a sponge form was attached, in addition to the felt comprising polyglycolic acid, to the suturing member, the intrapulmonary pressure at which air leakage was observed was 54.17±12.42 cmH₂O (mean±SD). This value is significantly greater (P = 0.0002) than that of Comparative Example A (12±6.78 cmH₂O) where nothing was attached to the suturing member. This value is also significantly greater (P = 0.005) than that of Comparative Example B (31.3±6.57 cmH₂O) where only the felt comprising polyglycolic acid was attached to the suturing member.

As described above, air leakage from the lung was significantly reduced when the alginic acid-Ca/Na salt sheet in a sponge form was attached, in addition to the felt comprising polyglycolic acid, to the suturing member, compared to the case where nothing was attached to the suturing member or where only the felt comprising polyglycolic acid was attached thereto.

### Example 2

### (1) Production of a suture reinforcement material for an automatic suturing device

The suture reinforcement material for an automatic suturing device of the present invention was produced in the following manner.

### • Production method

(i) An aqueous sodium alginate solution (2%, 10 mL) was poured into a plastic tray (5.3 x 8.3 cm²).
(ii) The resulting product was frozen at a temperature of -80°C.
(iii) The tray was placed in a freeze dryer and vacuum freeze-dried (24 hours).
(iv) The obtained sponge was cut into a size of 3.5 x 7.0 cm.
(v) The sodium alginate sheet in a sponge form was superimposed on a felt comprising polyglycolic acid ("NEOVEIL" (product name); GUNZE LIMITED) and having almost the same size.
(vi) The neoveil part of Neoveil, tube type (produced by GUNZE LIMITED), was replaced with the sheet material obtained above to produce the suture reinforcement material for an automatic suturing device of the present invention. Specifically, the sheet material obtained in (v) was cut into a rectangle, and a rectangular-shaped stretch knit material comprising nylon and polyurethane was superimposed thereon. Both ends of the sheet material and stretch knit material superimposed on each other were stitched together with a thread to form a tubular shape (tube shape) so as to be attached to the automatic suturing device, thereby producing a suture reinforcement material for an automatic suturing device of the present invention.

### (2) Suturing and dissection of biological tissue using suture reinforcement material for an automatic suturing device

In the same manner as in Example 1, the suture reinforcement material for an automatic suturing device of the present invention produced in (1) above was attached to an automatic suturing device and applied to a dog's lung. The lung was sutured using the automatic suturing device, then sprayed with a 0.6% aqueous calcium gluconate solution, and maintained for 10 seconds. Subsequently, the lung was dissected and sprayed again with a 0.6% aqueous calcium gluconate solution. The intrapulmonary pressure at which air leakage started to occur was measured. The experiment was performed with n = 3.

### • Results

Fig. 2 shows the results of Example 1, and the results of a significance test. Even in a case where sodium alginate was used as the hydrophilic polymer, and an aqueous calcium gluconate solution was sprayed, air leakage from the lung was significantly reduced, compared to the case where nothing was attached to the suturing member or where only the felt comprising polyglycolic acid was attached thereto.

### Example 3

### (1) Production of a suture reinforcement material for an automatic suturing device

The suture reinforcement material for an automatic suturing device of the present invention was produced in the following manner.

### • Production method

(i) An aqueous gelatin solution (1%, 10 mL) was poured into a plastic tray (5.3 x 8.3 cm²).
(ii) "NEOVEIL" (4.5 x 7 cm²) was placed in an aqueous solution and sufficiently immersed.
(iii) The resulting product was frozen at a temperature of -80°C.
(iv) The tray was placed in a freeze dryer and vacuum freeze-dried (24 hours).
(v) Thermal crosslinking of the sponge was performed (140°C, 3 hours) in a vacuum dryer.
(vi) The neoveil part of Neoveil, tube type (produced by GUNZE LIMITED), was replaced with the sheet material obtained above to produce the suture reinforcement material for an automatic suturing device of the present invention. Specifically, the sheet material obtained in (v) was cut into a rectangle, and a rectangular-shaped stretch knit material comprising nylon and polyurethane was superimposed thereon. Both ends of the sheet material and stretch knit material superimposed on each other were stitched together with a thread to form a tubular shape (tube shape) to be attached to the automatic suturing device, thereby producing a suture reinforcement material for an automatic suturing device of the present invention.

### (2) Suturing and dissection of biological tissue using a suture reinforcement material for an automatic suturing device

In the same manner as in Example 1, the suture reinforcement material for an automatic suturing device of the present invention produced in (1) above was attached to an automatic suturing device and applied to a dog's lung. Then, the intrapulmonary pressure at which air leakage started to occur was measured. The experiment was performed with n = 3.

### • Results

Fig. 2 shows the results of Example 1, and the results of a significance test. Even in a case where gelatin was used as the hydrophilic polymer, air leakage from the lung was significantly reduced, compared to the case where nothing was attached to the suturing member or where only the felt comprising polyglycolic acid was attached thereto.

## Claims

1. A suture reinforcement material for an automatic suturing device, comprising a sheet (A) containing at least one biodegradable and bioabsorbable material selected from the group consisting of aliphatic polyesters, polyester ethers, and copolymers thereof, wherein the suture reinforcement material further comprises a calcium ion source (C), and wherein the calcium ion source is at least one calcium salt selected from the group consisting of polysaccharides and saccharic acids,
the sheet (A) having on at least one surface thereof a layer (B) containing a hydrophilic polymer wherein the hydrophilic polymer is at least one member selected from the group consisting of alginic acid and alginates.

2. The suture reinforcement material for an automatic suturing device according to claim 1, wherein the layer containing a hydrophilic polymer is porous.

3. The suture reinforcement material for an automatic suturing device according to any one of claims 1 to 2, wherein the aliphatic polyester is polyglycolic acid, polylactic acid, polycaprolactone, polyvalerolactone, or a copolymer of these compounds.

4. The suture reinforcement material for an automatic suturing device according to any one of claims 1 to 3, wherein the polyester ether is poly-1,4-dioxanone-2-one, poly-1,5-dioxepane-2-one, or a copolymer of ethylene glycol and aliphatic polyester.

5. The suture reinforcement material for an automatic suturing device according to any one of claims 1 to 4, wherein the material is in a tubular shape.

6. The suture reinforcement material for an automatic suturing device according to claim 5, further comprising a stretch knitted or woven fabric (D), the stretch knitted or woven fabric (D) being joined with the sheet (A).

7. A method for producing a suture reinforcement material according to claim 1 comprising: a step of forming the layer (B) on at least one surface of the sheet (A).

8. A method according to claim 7 for producing a suture reinforcement material according to claim 1 comprising: a step of further forming a calcium ion source (C) thereon.

9. A method according to claim 7 for producing a suture reinforcement material according to claim 1 comprising: a step of forming a calcium ion source (C) on at least one surface of the sheet (A), before the step of forming the layer (B) thereon.

10. A method according to claim 7 for producing a suture reinforcement material according to claim 1 wherein the layer (B) and a calcium ion source (C) are mixed, and the resulting mixture is applied to at least one surface of the sheet (A).

11. The method according to claims 7 to 10 comprising: a step of forming the layer (B) into a porous body.

## Patentansprüche

1. Ein Nahtverstärkungsmaterial für eine automatische Nähvorrichtung, umfassend ein Bahnenmaterial (A), das mindestens ein biologisch abbaubares und biologisch absorbierbares Material enthält, ausgewählt aus der Gruppe bestehend aus aliphatischen Polyestern, Polyesterethern und Copolymeren davon, wobei das Nahtverstärkungsmaterial darüber hinaus eine Calciumionenquelle (C) umfasst, und wobei die Calciumionenquelle mindestens ein Calciumsalz ist, ausgewählt aus der Gruppe bestehend aus Polysacchariden und Saccharidsäuren,
wobei das Bahnenmaterial (A) auf mindestens einer Oberfläche davon eine Schicht (B) aufweist, die ein hydrophiles Polymer enthält, wobei das hydrophile Polymer mindestens eines ist, ausgewählt aus der Gruppe bestehend aus Alginsäure und Alginaten.

2. Das Nahtverstärkungsmaterial für eine automatische Nähvorrichtung gemäß Anspruch 1, wobei die Schicht, die ein hydrophiles Polymer enthält, porös ist.

3. Das Nahtverstärkungsmaterial für eine automatische Nähvorrichtung gemäß einem der Ansprüche 1 bis 2, wobei der aliphatische Polyester Polyglycolsäure, Polymilchsäure, Polycaprolacton, Polyvalerolacton oder ein Copolymer dieser Verbindungen ist.

4. Das Nahtverstärkungsmaterial für eine automatische Nähvorrichtung gemäß einem der Ansprüche 1 bis 3, wobei der Polyesterether Poly-1,4-dioxanon-2-on, Poly-1,5-dioxepan-2-on oder ein Copolymer aus Ethylenglycol und aliphatischem Polyester ist.

5. Das Nahtverstärkungsmaterial für eine automatische Nähvorrichtung gemäß einem der Ansprüche 1 bis 4, wobei das Material eine tubuläre Gestalt aufweist.

6. Das Nahtverstärkungsmaterial für eine automatische Nähvorrichtung gemäß Anspruch 5, weiter umfassend einen stretch gestrickten oder gewobenen Stoff (D), wobei der stretch gestrickte oder gewobene Stoff (D) mit dem Bahnenmaterial (A) verbunden ist.

7. Ein Verfahren zur Herstellung eines Nahtverstärkungsmaterials gemäß Anspruch 1, umfassend: einen Schritt des Bildens der Schicht (B) auf mindestens einer Oberfläche des Bahnenmaterials (A).

8. Ein Verfahren gemäß Anspruch 7 zur Herstellung eines Nahtverstärkungsmaterials gemäß Anspruch 1, umfassend: einen Schritt des weiteren Bildens einer Calciumionenquelle (C) darauf.

9. Ein Verfahren gemäß Anspruch 7 zur Herstellung eines Nahtverstärkungsmaterials gemäß Anspruch 1, umfassend: einen Schritt des Bildens einer Calciumionenquelle (C) auf mindestens einer Oberfläche des Bahnenmaterials (A) vor dem Schritt des Bildens der Schicht (B) darauf.

10. Ein Verfahren gemäß Anspruch 7 zur Herstellung eines Nahtverstärkungsmaterials gemäß Anspruch 1, wobei die Schicht (B) und eine Calciumionenquelle (C) gemischt werden und das so erhaltene Gemisch auf mindestens eine Oberfläche des Bahnenmaterials (A) aufgetragen wird.

11. Das Verfahren gemäß den Ansprüchen 7 bis 10, umfassend: einen Schritt des Formens der Schicht (B) zu einem porösen Körper.

## Revendications

1. Matériau de renfort de suture pour dispositif de suturage automatique, comprenant une feuille (A) contenant au moins un matériau biodégradable et bioabsorbable choisi dans le groupe constitué par les polyesters aliphatiques, les éthers de polyester, et des copolymères de ceux-ci, dans lequel le matériau de renfort de suture comprend en outre une source d'ions de calcium (C), et dans lequel la source d'ions de calcium est au moins un sel de calcium choisi dans le groupe constitué par les polysaccharides et les acides sacchariques,
la feuille (A) ayant sur au moins une de ses surfaces une couche (B) contenant un polymère hydrophile où le polymère hydrophile est au moins un élément choisi dans le groupe constitué par l'acide alginique et les alginates.

2. Matériau de renfort de suture pour dispositif de suturage automatique selon la revendication 1, dans lequel la couche contenant un polymère hydrophile est poreuse.

3. Matériau de renfort de suture pour dispositif de suturage automatique selon l'une quelconque des revendications 1 à 2, dans lequel le polyester aliphatique est l'acide polyglycolique, l'acide polylactique, la polycaprolactone, la polyvalérolactone, ou un copolymère de ces composés.

4. Matériau de renfort de suture pour dispositif de suturage automatique selon l'une quelconque des revendications 1 à 3, dans lequel l'éther de polyester est une poly-1,4-dioxanon-2-one, une poly-1,5-dioxépan-2-one, ou un copolymère d'éthylène glycol et d'un polyester aliphatique.

5. Matériau de renfort de suture pour dispositif de suturage automatique selon l'une quelconque des revendications 1 à 4, dans lequel le matériau est sous une forme tubulaire.

6. Matériau de renfort de suture pour dispositif de suturage automatique selon la revendication 5, comprenant en outre un tissu à mailles extensibles ou tissé (D), le tissu à mailles extensibles ou tissé (D) étant joint à la feuille (A).

7. Méthode de production d'un matériau de renfort de suture selon la revendication 1, comprenant : une étape de formation de la couche (B) sur au moins une surface de la feuille (A).

8. Méthode selon la revendication 7 de production d'un matériau de renfort de suture selon la revendication 1, comprenant : une étape de formation supplémentaire d'une source d'ions de calcium (C) sur celui-ci.

9. Méthode selon la revendication 7 de production d'un matériau de renfort de suture selon la revendication 1, comprenant : une étape de formation d'une source d'ions de calcium (C) sur au moins une surface de la feuille (A), avant l'étape de formation de la couche (B) sur celui-ci.

10. Méthode selon la revendication 7 de production d'un matériau de renfort de suture selon la revendication 1 dans laquelle la couche (B) et une source d'ions de calcium (C) sont mélangées, et le mélange obtenu est appliqué sur au moins une surface de la feuille (A).

11. Méthode selon les revendications 7 à 10 comprenant : une étape de formation de la couche (B) dans un corps poreux.
